(19) Europäisches Patentamt
European Patent Office
Office européen des brevets

(11) Numéro de publication : **0 018 931**
**B1**

(12) **FASCICULE DE BREVET EUROPÉEN**

(45) Date de publication du fascicule du brevet : **10.09.86**

(51) Int. Cl.⁴ : **C 07 D263/58**

(21) Numéro de dépôt : **80420052.5**

(22) Date de dépôt : **07.05.80**

(54) **Préparation de benzoxazolone.**

(30) Priorité : **08.05.79 FR 7912139**

(43) Date de publication de la demande : **12.11.80 Bulletin 80/23**

(45) Mention de la délivrance du brevet : **10.09.86 Bulletin 86/37**

(84) Etats contractants désignés : **AT BE CH DE FR GB IT LI LU NL SE**

(56) Documents cités : **FR-A- 1 269 067**
**Le dossier contient des informations techniques présentées postérieurement au dépôt de la demande et ne figurant pas dans le présent fascicule.**

(73) Titulaire : **RHONE-POULENC AGROCHIMIE**
**14-20, rue Pierre Baizet**
**F-69009 Lyon (FR)**

(72) Inventeur : **Querou, Yvon**
**2, Rue Zilina**
**F-92000-Nanterre (FR)**

(74) Mandataire : **Brachotte, Charles et al**
**RHONE-POULENC AGROCHIMIE BP 9163-09**
**F-69263 Lyon Cedex 1 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 018 931 B1

**Description**

La présente invention concerne un procédé de préparation de composé hétérocyclique à partir de phénol. Plus spécifiquement, l'invention concerne un procédé de préparation de benzoxazolone à partir de chlorophénol.

La benzoxazolone a pour formule :

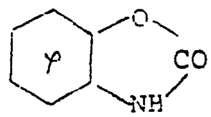

Il s'agit d'un produit intermédiaire utile pour la synthèse d'autres produits, par exemple l'insecticide connu sous le nom de phosalone. Cette benzoxazolone est quelquefois appelée, notamment en langue anglaise, benzoxazolinone. On sait préparer la benzoxazolone par des voies assez diverses partant de réactifs plus ou moins élaborés. Un but de l'invention est de fournir un procédé simple de préparation de la benzoxazolone à partir de réactifs simples, et plus particulièrement à partir d'orthochlorophénol. Il a donc été trouvé un procédé de préparation de benzoxazolone à partir d'orthochlorophénol caractérisé en ce que (dans une première étape) un mélange d'orthochlorophénol et d'urée est chauffé sous pression d'ammoniac et puis, que (dans une deuxième étape) le mélange réactionnel obtenu au cours de la première étape est chauffé sous pression atmosphérique, de préférence en présence d'eau.

Selon un mode avantageux de réalisation de l'invention la première étape est réalisée en présence de catalyseur métallique ; comme catalyseur on utilise avantageusement un catalyseur à base de cuivre ; comme catalyseurs utilisables, on peut citer, les sels cuivreux ou cuivriques, notamment les halogénures, sulfates, phosphates, acétates, propionates, acétylacétonates (le chlorure cuivreux est préféré), ainsi que les oxydes (notamment l'oxyde cuivreux) et le cuivre métallique. La quantité de catalyseur comprise dans le milieu réactionnel est généralement comprise entre 0,5 et 20 % en poids par rapport à l'orthochlorophénol, de préférence comprise entre 2 et 10 %. Des quantités sortant de ces limites peuvent, cependant être utilisées, sans toutefois que cela présente un avantage économique important.

La première étape réactionnelle est donc effectuée sous pression d'ammoniac. Autrement dit, il s'agit d'un chauffage d'un milieu réactionnel liquide, surmonté par une atmosphère sous pression contenant de l'ammoniac. Il s'agit de préférence de $NH_3$ ajouté, sous pression, dans l'atmosphère surmontant le milieu réactionnel, mais il est aussi possible d'obtenir une telle atmosphère sous pression d'ammoniac en laissant se décomposer l'urée. La pression totale est généralement comprise entre 1 et 60 bars (pressions relatives), de préférence entre 3 et 40 bars. Des pressions supérieures, allant par exemple jusqu'à 150 bars, peuvent être aussi utilisées, mais sans beaucoup d'intérêt économique. L'introduction d'eau dans le milieu réactionnel constitue une variante de l'invention qui a notamment pour effet de décomposer partiellement l'urée et de favoriser la formation d'ammoniac, ce qui peut contribuer à augmenter la pression d'ammoniac régnant sur le milieu réactionnel. La teneur en eau du milieu réactionnel est habituellement inférieure à 20 %, de préférence inférieure à 10 % (pourcentages pondéraux par rapport à l'ensemble du milieu réactionnel).

Dans cette atmosphère contenant de l'ammoniac et surmontant le milieu réactionnel lors de la première étape, l'ammoniac ($NH_3$) se trouve généralement à une pression partielle supérieure à 50 % de la pression totale, de préférence supérieure à 90 % de cette pression. Cet ammoniac provient le plus souvent soit d'une alimentation extérieure, soit d'une introduction préalable dans le réacteur, soit d'une décomposition de l'urée, soit d'une conjonction de plusieurs de ces facteurs. Le rapport molaire de l'urée mise en œuvre au cours de la réaction par rapport à l'orthochlorophénol mis en œuvre est habituellement compris entre 1 et 15, de préférence entre 1,2 et 8.

L'urée présente dans le milieu réactionnel peut éventuellement être préparée *in situ* par réaction de $CO_2$ avec l'ammoniac. La température du milieu réactionnel est généralement comprise, dans la première étape, entre 100 et 250 °C, de préférence entre 140 et 230 °C.

Cette première étape réactionnelle peut être effectuée en présence de solvants minéraux ou organiques, mais l'on préfère généralement opérer en masse. Cette masse est normalement liquide à la température réactionnelle (elle peut être non liquide à température ambiante).

La durée de cette première étape réactionnelle est évidemment variable selon les conditions opératoires. L'homme de l'art pourra par de simples essais de routine déterminer la durée optimale ; généralement on poursuit cette étape réactionnelle jusqu'à ce que la teneur en orthocholorophénol ne varie plus substantiellement ou, autrement dit, jusqu'à ce que le taux de transformation de l'orthochlorophénol ait sensiblement atteint son maximum, la simple dégradation thermique et/ou chimique étant mise à part.

Comme cela a déjà été indiqué, le procédé de l'invention comprend 2 étapes réactionnelles ; ces 2 étapes sont effectivement distinctes notamment par le fait que la première est effectuée sous pression alors que la seconde est simplement effectuée sous pression atmosphérique. Cependant, hormis cette distinction, ces 2 étapes sont assez voisines sur le plan pratique et elles peuvent être effectuées l'une à la suite de l'autre, simplement par un petit changement de conditions opératoires, mais sans qu'il soit

vraiment nécessaire, entre les 2 étapes réactionnelles, de changer le milieu réactionnel de réacteur ou de le soumettre à des traitements particuliers. Cela fait ressortir que malgré la présence apparente de 2 étapes réactionnelles, le procédé de l'invention est d'une grande simplicité et d'une grande commodité de mise en œuvre. Cette simplicité et cette commodité sont pratiquement aussi grandes que s'il s'agissait d'un procédé ne comportant strictement qu'une étape réactionnelle.

La seconde étape réactionnelle s'effectue sous pression atmosphérique, de préférence sous atmosphère libre. Etant donné la volatilité de l'ammoniac, le chauffage sous pression atmosphérique est équivalent à un chauffage en absence totale ou presque totale d'ammoniac. Un peu d'ammoniac peut cependant être présent, notamment sous l'effet d'une certaine décomposition de l'urée, mais même dans ce cas, l'ammoniac s'échappe du milieu à chaud.

La température réactionnelle au cours de cette seconde étape est généralement comprise entre 80 et 220 °C de préférence entre 110 et 190 °C.

On préfère opérer cette seconde étape réactionnelle en présence d'eau. Toutefois étant donné la température et la pression, l'eau a tendance à s'évaporer plus ou moins rapidement en sorte que, selon une variante, on alimente le milieu réactionnel en eau liquide de manière continue et, éventuellement, on récupère et condense la vapeur d'eau issue du milieu réactionnel. Selon de telles modalités, il est avantageux d'alimenter en eau le milieu réactionnel avec un débit horaire inférieur à 20 % en poids de ce milieu réactionnel.

En fin de réaction, on isole la benzoxazolone par tout moyen connu en soi. Selon une modalité opérationnelle très avantageuse, on précipite la benzoxazolone par de l'eau éventuellement acidifiée ; la benzoxazolone peut être purifiée par tout moyen connu en soi, par exemple par recristallisation, ou lavage avec des solvants organiques.

Le procédé de l'invention est spécialement avantageux en raison des bons résultats obtenus, tant ce qui concerne le taux de transformation de l'orthochlorophénol que les rendements en benzoxazolone et que la simplicité et la commodité de mise en œuvre.

Les exemples suivants, donnés à titre non limitatif, illustrent l'invention et montrent comment elle peut être mise en œuvre.

Dans ces exemples T T désigne le taux de transformation de l'orthochlorophénol et R T désigne le rendement en benzoxazolone par rapport à l'orthochlorophénol transformé.

### Exemple 1

Dans un autoclave en acier inoxydable de 1,5 l muni d'un système d'agitation, on charge :

257 g d'orthochlorophénol
300 g d'urée
19,8 g de $Cu_2Cl_2$

On purge à l'aide d'ammoniac. On chauffe l'autoclave à 170 °C puis on met sous pression constante de 22 bars d'ammoniac (pression relative). On poursuit le chauffage pendant 6 heures au cours desquelles il s'est consommé 69 g de $NH_3$. On refroidit jusqu'à 110 °C, dégaze l'autoclave et on le munit d'un réfrigérant descendant relié à ce réacteur par l'intermédiaire d'une tubulure coudée. La température est portée à 125 °C ; la pression est égale à la pression atmosphérique car le milieu réactionnel est en connection avec l'atmosphère libre par le moyen du réfrigérant descendant. On ajoute de l'eau au milieu réactionnel à raison de 65 $cm^3$/h tout en chauffant et distillant pendant 2 heures à 140 °C, puis 2 heures à 150 °C. On refroidit à 120 °C, on ajoute 0,8 l d'eau. La benzoxazolone précipite ; on laisse le milieu réactionnel une nuit sous agitation et rajoute encore 0,15 l d'une solution aqueuse d'acide sulfurique à 3 moles/l. La benzoxazolone est filtrée puis lavée et recristallisée. On obtient ainsi la benzoxazolone avec un R T de 74,5 % et un T T de 94,5 %.

### Exemple 2

On reproduit l'exemple 1 en n'utilisant que 9,9 g de $Cu_2Cl_2$ (au lieu de 19,8 g). Il se consomme 60 g de $NH_3$. On obtient la benzoxazolone avec un R T de 79,5 % et un T T de 84,5 %.

### Exemple 3

On reproduit l'exemple 1, mais en utilisant les quantités de réactifs suivantes :

385,5 g d'orthochlorophénol
270 g d'urée
29,7 g de $Cu_2Cl_2$

Au cours de la réaction, il se consomme 93 g de $NH_3$. On obtient la benzoxazolone avec un R T de 67 % et un T T de 93,5 %.

Exemple 4

Dans un autoclave de 1,5 l, on charge :

257 g d'orthochlorophénol
300 g d'urée
19,8 g de $Cu_2Cl_2$
68,1 g de $NH_3$

On chauffe à 170 °C pendant 8 heures ; la pression s'établit initialement à 26 bars, puis descend progressivement jusqu'à 10 bars en fin de réaction. On refroidit à 120 °C, dégaze, puis on coule lentement 30 g d'eau et chauffe 2 heures à 140 °C, puis 2 heures à 150 °C tout en distillant à pression atmosphérique. Après traitement, comme à l'exemple 1, on obtient la benzoxazolone avec un R T de 70 % et un T T de 96 %.

Exemple 5

On reproduit l'exemple 4, mais avec 9,9 g de $Cu_2Cl_2$ (au lieu de 19,8 g) ; on obtient ainsi la benzoxazolone avec un R T de 70,5 % et un T T de 80,5 %.

Exemple 6

Dans un autoclave en acier inoxydable de 1 l muni d'un système d'agitation, on charge :

102,8 g d'orthochlorophénol
240 g d'urée
7,92 g de $Cu_2Cl_2$

On ferme l'autoclave, purge à l'aide de $NH_3$ et on charge 51 g de $NH_3$. On chauffe à 170 °C pendant 8 heures. La pression se stabilise initialement à 38 bars (pression relative) puis descend progressivement jusqu'à 24 bars en fin de réaction.

On refroidit, ouvre l'utoclave, ajoute 16 g d'eau, et chauffe ce mélange à l'air libre et sous pression atmosphérique à environ 135 °C pendant 4 heures.

En fin de réaction, on ajoute à ce milieu réactionnel 600 cm³ d'une solution aqueuse N d'acide sulfurique. On filtre, on essore le précipité, on obtient ainsi la benzoxazolone avec un T T de 99 % et un R T de 78,6 %.

Exemple 7

Dans un autoclave de 125 cm³ on charge :

12,85 g d'orthochlorophénol
0,715 g de $Cu_2O$
9 g d'urée
13,6 g de $NH_3$

On chauffe 8 heures à 140 °C. La pression initialement de 32 bars descend progressivement à 17 bars. On refroidit à 125 °C et ajoute 2 cm³ d'eau, puis sans distiller on chauffe à pression atmosphérique 1 heure à 130 °C et 2 heures à 150 °C.

Après traitement habituel, on obtient la benzoxazolone avec un R T de 46 % et un T T de 95,5 %.

Exemple 8

Dans un autoclave de 125 cm³, on charge :

30 g d'urée
12,85 g d'orthochlorophénol
0,99 g de $Cu_2Cl_2$
0,68 g de $NH_3$

On chauffe 8 heures à 170 °C. La pression s'établit à 10 bars et descend jusqu'à 9 bars. On poursuit les opérations comme à l'exemple 7 et on obtient la benzoxazolone avec un R T de 41,5 % et un T T de 89 %.

Exemple 9

Dans un autoclave de 1 l, on charge :

102,8 g d'orthochlorophénol
240 g d'urée
7,92 g de $Cu_2Cl_2$
14,4 g d'eau

On purge à l'azote et chauffe 8 heures à 170 °C. L'atmosphère sous pression d'ammoniac est ainsi obtenue par décomposition de l'urée en présence d'eau. Cette pression s'établit à 40 bars et se maintient à cette valeur. On refroidit, dégaze l'autoclave, on chauffe tout en distillant à pression atmosphérique 2 heures à 130 °C puis 2 heures à 150 °C.

Après traitements habituels, on obtient la benzoxazolone avec un R T de 57,5 % et un T T de 95 %.

Exemple 10

Dans un autoclave de 140 $cm^3$ revêtu intérieurement de polytétrafluoroéthylène, on charge :

24 g d'orthochlorophénol
28 g d'urée
2,5 g de $CuCl_2$
6,35 g de $NH_3$.

On ferme l'autoclave, purge à l'aide de $NH_3$. On chauffe à 170 °C pendant 6 heures. La pression se stabilise initialement à environ 26 bars (pression relative) puis descend progressivement jusqu'à environ 10 bars en fin de réaction. On refroidit à 120 °C, ouvre l'autoclave, et transfère le contenu de l'autoclave dans un ballon de 250 $cm^3$ sous pression atmosphérique muni d'un système de distillation semblable à celui de l'exemple 1. Dans ce ballon on ajoute lentement 3 $cm^3$ d'eau ; on chauffe alors 2 heures à 140 °C puis 2 heures à 150 °C en ajoutant régulièrement de l'eau à raison de 4,5 $cm^3$/h tout au long de la distillation. On refroidit à nouveau à 120 °C, ajoute 80 $cm^3$ d'eau, refroidit à 20 °C, ajoute 15 $cm^3$ d'une solution aqueuse d'acide sulfurique 6 N, ajoute 50 $cm^3$ d'acétate d'éthyle. On décante et sépare la phase aqueuse qu'on lave à l'aide d'acétate d'éthyle. Dans la phase organique on obtient de la benzoxazolone avec un R T de 30 % et un T T de 80 %.

On a également obtenu la benzoxazolone en présence de $FeCl_2$, $NiCl_2$ et Ni.

Exemple 11

On opère comme à l'exemple 10 mais avec les modifications suivantes :
On charge

15,8 g d'orthochlorophénol
59 g d'urée
1,23 g de chlorure cuivreux
6,35 g de $NH_3$.

On chauffe 6 h à 210 °C. La pression se stabilise initialement à environ 24 bars (pression relative) puis descend progressivement jusqu'à environ 10 bars en fin de réaction.

Après transfert dans le ballon sous pression atmosphérique, on chauffe pendant 2 h à 170 °C puis 2 h à 190 °C.

On obtient la benzoxazolone avec un R T de 59 % et un T T de 98 %.

**Revendications**

1. Procédé de préparation de benzoxazolone, caractérisé en ce qu'un mélange d'orthochlorophénol et d'urée est chauffé sous pression d'ammoniac jusqu'à ce que le degré de conversion de l'orthochlorophénol se soit stabilisé, puis que ce mélange est chauffé sous pression atmosphérique.

2. Procédé de préparation de benzoxazolone selon la revendication 1, caractérisé en ce que le chauffage du mélange d'orthochlorophénol et d'urée sous pression d'ammoniac est effectué à une pression comprise entre 1 et 60 bars.

3. Procédé de préparation de benzoxazolone selon la revendication 2, caractérisé en ce que la pression est comprise entre 3 et 40 bars.

4. Procédé de préparation de benzoxazolone selon l'une des revendications 2 ou 3, caractérisé en ce que la pression partielle d'ammoniac est supérieure à 50 % de la pression totale.

5. Procédé de préparation de benzoxazolone selon l'une des revendications 2 à 4, caractérisé en ce que la pression partielle d'ammoniac est supérieure à 90 % de la pression totale.

6. Procédé selon l'une des revendications 1 à 5, caractérisé en ce que le rapport molaire de l'urée mise en œuvre, par rapport à l'orthochlorophénol mis en œuvre, est compris entre 1 et 15.

7. Procédé selon la revendication 6, caractérisé en ce que le rapport urée/orthochlorophénol est compris entre 1,2 et 8.

8. Procédé selon l'une des revendications 1 à 7, caractérisé en ce que la température du milieu réactionnel lors de la première étape réactionnelle est comprise entre 100 et 250 °C.

9. Procédé selon la revendication 8, caractérisé en ce que la température du milieu réactionnel lors de la première étape réactionnelle est comprise entre 140 et 230 °C.

10. Procédé selon l'une des revendications 1 à 9, caractérisé en ce que la température du milieu réactionnel lors de la deuxième étape réactionnelle est comprise entre 80 et 220 °C.

11. Procédé selon la revendication 10, caractérisé en ce que la température du milieu réactionnel lors de la deuxième étape réactionnelle, est comprise entre 110 et 190 °C.

12. Procédé selon la revendication 11 caractérisé en ce que, au moins lors de la deuxième étape réactionnelle, la réaction est effectuée en présence d'eau.

13. Procédé selon l'une des revendications 1 à 12, caractérisé en ce qu'on opère en présence d'un catalyseur métallique.

14. Procédé selon la revendication 13, caractérisé en ce que le catalyseur est à base de cuivre.

15. Procédé selon la revendication 14, caractérisé en ce que le catalyseur est un sel cuivreux.

16. Procédé selon la revendication 15, caractérisé en ce que le catalyseur est du chlorure cuivreux.

17. Procédé selon l'une des revendications 13 à 16, caractérisé en ce que la teneur en catalyseur du milieu réactionnel est comprise entre 0,5 et 20 % en poids par rapport à l'orthochlorophénol.

18. Procédé selon la revendication 17 caractérisé en ce que la teneur en catalyseur est comprise entre 2 et 10 % en poids par rapport à l'orthochlorophénol.

19. Procédé de préparation de benzoxazolone, caractérisé en ce que un mélange d'orthochlorophénol et d'urée est chauffé en présence d'un sel cuivreux sous pression d'ammoniac, la pression étant comprise entre 10 et 60 bars et la pression partielle d'ammoniac étant supérieure à 90 % de la pression totale, puis que ce mélange est chauffé sous pression atmosphérique.

**Claims**

1. Process for the preparation of benzoxazolone, characterized in that a mixture of ortho-chlorophenol and urea is heated under ammonia pressure until the degree of conversion of the ortho-chlorophenol has stabilized and that then this mixture is heated under atmospheric pressure.

2. Process for the preparation of benzoxazolone according to Claim 1, characterized in that the heating of the mixture of ortho-chlorophenol and urea under ammonia pressure is carried out at a pressure between 1 and 60 bars.

3. Process for the preparation of benzoxazolone according to Claim 2, characterized in that the pressure is between 3 and 40 bars.

4. Process for the preparation of benzoxazolone according to one of Claims 2 and 3, characterized in that the partial pressure of ammonia is more than 50 % of the total pressure.

5. Process for the preparation of benzoxazolone according to one of Claims 2 to 4, characterized in that the partial pressure of ammonia is more than 90 % of the total pressure.

6. Process according to one of Claims 1 to 5, characterized in that the molar ratio of the urea used to the ortho-chlorophenol used is between 1 and 15.

7. Process according to Claim 6, characterized in that the ratio urea/ortho-chlorophenol is between 1.2 and 8.

8. Process according to one of Claims 1 to 7, characterized in that the temperature of the reaction medium in the first reaction step is between 100 and 250 °C.

9. Process according to Claim 8, characterized in that the temperature of the reaction medium in the first reaction step is between 140 and 230 °C.

10. Process according to one of Claims 1 to 9, characterized in that the temperature of the reaction medium in the second reaction step is between 80 and 220 °C.

11. Process according to Claim 10, characterized in that the temperature of the reaction medium in the second reaction step is between 110 and 190 °C.

12. Process according to Claim 11, characterized in that the reaction is carried out in the presence of water, at least in the second reaction step.

13. Process according to one of Claims 1 to 12, characterized in that the reaction is carried out in the presence of a metal catalyst.

14. Process according to Claim 13, characterized in that the catalyst is based on copper.

15. Process according to Claim 14, characterized in that the catalyst is a cuprous salt.

16. Process according to Claim 15, characterized in that the catalyst is cuprous chloride.

17. Process according to one of Claims 13 to 16, characterized in that the proportion of catalyst in the reaction medium is between 0.5 and 20 % by weight, relative to the ortho-chlorophenol.

18. Process according to Claim 17, characterized in that the proportion of catalyst is between 2 and 10 % by weight, relative to the ortho-chlorophenol.

19. Process for the preparation of benzoxazolone, characterized in that a mixture of ortho-chlorophenol and urea is heated in the presence of a cuprous salt under ammonia pressure, the pressure being between 10 and 60 bars and the partial pressure of ammonia being more than 90 % of the total pressure, and that then this mixture is heated under atmospheric pressure.

**Patentansprüche**

1. Verfahren zur Herstellung von Benzoxazolon, dadurch gekennzeichnet, daß man ein Gemisch von Orthochlorphenol und Harnstoff unter Ammoniakdruck erhitzt, bis der Umwandlungsgrad des Orthochlorphenols sich stabilisiert hat, und daß man dieses Gemisch dann unter Atmosphärendruck erhitzt.

2. Verfahren zur Herstellung von Benzoxazolon gemäß Anspruch 1, dadurch gekennzeichnet, daß das Erhitzen des Gemisches aus Orthochlorphenol und Harnstoff unter Ammoniakdruck bei einem Druck zwischen 1 und 60 bar bewirkt wird.

3. Verfahren zur Herstellung von Benzoxazolon gemäß Anspruch 2, dadurch gekennzeichnet, daß der Druck zwischen 3 und 40 bar beträgt.

4. Verfahren zur Herstellung von Benzoxazolon gemäß einem der Ansprüche 2 oder 3, dadurch gekennzeichnet, daß der Teildruck von Ammoniak oberhalb 50 % des Gesamtdrucks liegt.

5. Verfahren zur Herstellung von Benzoxazolon gemäß einem der Ansprüche 2 bis 4, dadurch gekennzeichnet, daß der Teildruck von Ammoniak oberhalb 90 % des Gesamtdrucks liegt.

6. Verfahren gemäß einem der Ansprüche 1 bis 5, dadurch gekennzeichnet, daß das Molverhältnis des eingesetzten Harnstoffs in bezug auf eingesetztes Orthochlorphenol zwischen 1 und 15 beträgt.

7. Verfahren gemäß Anspruch 6, dadurch gekennzeichnet, daß das Verhältnis Harnstoff/Orthochlorphenol zwischen 1,2 und 8 liegt.

8. Verfahren gemäß einem der Ansprüche 1 bis 7, dadurch gekennzeichnet, daß die Temperatur des Reaktionsmilieus während der ersten Reaktionsstufe zwischen 100 und 250 °C beträgt.

9. Verfahren gemäß Anspruch 8, dadurch gekennzeichnet, daß die Temperatur des Reaktionsmilieus während der ersten Reaktionsstufe zwischen 140 und 230 °C liegt.

10. Verfahren gemäß einem der Ansprüche 1 bis 9, dadurch gekennzeichnet, daß die Temperatur des reaktionsmilieus während der zweiten Reaktionsstufe zwischen 80 und 220 °C liegt.

11. Verfahren gemäß Anspruch 10, dadurch gekennzeichnet, daß die Temperatur des Reaktionsmilieus während der zweiten Reaktionsstufe zwischen 110 und 190 °C liegt.

12. Verfahren gemäß Anspruch 11, dadurch gekennzeichnet, daß wenigstens während der zweiten Reaktionsstufe die Reaktion in Gegenwart von Wasser bewirkt wird.

13. Verfahren gemäß einem der Ansprüche 1 bis 12, dadurch gekennzeichnet, daß man in Gegenwart eines Metallkatalysators arbeitet.

14. Verfahren gemäß Anspruch 13, dadurch gekennzeichnet, daß der Katalysator auf Basis von Kupfer ist.

15. Verfahren gemäß Anspruch 14, dadurch gekennzeichnet, daß der Katalysator ein Cuprosalz ist.

16. Verfahren gemäß Anspruch 15, dadurch gekennzeichnet, daß der Katalysator Cuprochlorid ist.

17. Verfahren gemäß den Ansprüchen 13 bis 16, dadurch gekennzeichnet, daß der Gehalt an Katalysator des Reaktionsmilieus zwischen 0,5 und 20 Gew.% in bezug auf das Orthochlorphenol beträgt.

18. Verfahren gemäß Anspruch 17, dadurch gekennzeichnet, daß der Gehalt an Katalysator zwischen 2 und 10 Gew.% in bezug auf das Orthochlorphenol liegt.

19. Verfahren zur Herstellung von Benzoxazolon, dadurch gekennzeichnet, daß ein Gemisch von Orthochlorphenol und Harnstoff in Gegenwart eines Kuprosalzes unter Ammoniakdruck erhitzt wird, wobei der Druck zwischen 10 und 60 bar beträgt und der Ammoniak-Teildruck oberhalb 90 % des Gesamtdrucks beträgt, daß man dieses Gemisch dann unter Atmosphärendruck erhitzt.